# EUROPEAN PATENT SPECIFICATION

(11) **EP 2 595 586 B1**
(45) Date of publication and mention of the grant of the patent: **04.09.2019**
(21) Application number: 11810348.0
(22) Date of filing: 20.07.2011
(51) Int. Cl.: A61F 9/007, A61B 3/10, A61B 3/113, A61B 90/00, A61B 17/00

(54) **SURFACE TRACKING AND MOTION COMPENSATING SURGICAL TOOL SYSTEM**
CHIRURGISCHES WERKZEUGSYSTEM MIT OBERFLÄCHENVERFOLGUNG UND BEWEGUNGSKOMPENSATION
SYSTÈME D'OUTIL CHIRURGICAL PERMETTANT LE SUIVI DE SURFACES ET LA COMPENSATION DE MOUVEMENTS

(30) Priority: 20.07.2010 US 365998 P
(43) Date of publication of application: 29.05.2013
(73) Proprietor: The Johns Hopkins University, Baltimore, MD 21218 (US)
(72) Inventor: KANG, Jin Ung, Ellicott City, Maryland 21043 (US); GEHLBACH, Peter, Hunt Valley, Maryland 21030 (US); TAYLOR, Russell H., Severna Park, Maryland 21146 (US); ZHANG, Kang, Catonsville, Maryland 21228 (US)
(74) Representative: dompatent von Kreisler Selting Werner - Partnerschaft von Patent- und Rechtsanwälten mbB
(86) International application number: PCT/US2011/044693
(87) International publication number: WO 2012/012540

(56) References cited:
- WO-A1-02/083003
- WO-A2-00/41611
- WO-A2-2007/109540
- US-A- 4 491 776
- US-A1- 2004 170 334
- US-A1- 2004 243 147
- US-A1- 2007 081 732
- US-A1- 2009 253 985
- KANG ZHANG ET AL: "A Surface Topology and Motion Compensation System for Microsurgery Guidance and Intervention Based on Common-Path Optical Coherence Tomography", IEEE TRANSACTIONS ON BIOMEDICAL ENGINEERING, IEEE SERVICE CENTER, PISCATAWAY, NJ, USA, vol. 56, no. 9, 1 September 2009 (2009-09-01), pages 2318-2321, XP011343041, ISSN: 0018-9294, DOI: 10.1109/TBME.2009.2024077

## Description

### BACKGROUND

### 1. Field of Invention

The field of the currently claimed embodiments of this invention relates to surgical tools and systems that incorporate the surgical tools, and more particularly to systems and surgical tools that have integrated surface tracking and motion compensation sensors.

### 2. Discussion of Related Art

Retinal surgery is one example of microsurgery. In current practice, retinal surgery is performed under an operating microscope with free-hand instrumentation. Human limitations include an inability to clearly view surgical targets, physiological hand tremor, and lack of tactile feedback in tool-to-tissue interactions. In addition, tool limitations, such as lack of proximity sensing or smart functions, are important factors that contribute to surgical risk and reduce the likelihood of achieving surgical goals. Current instruments do not provide physiological or even basic interpretive information. Surgical outcomes (both success and failure) are limited, in part, by technical hurdles that cannot be overcome by conventional instrumentation.

Microsurgery requires constant attention to and compensation for involuntary patient motion due to physiological processes such as breathing and cardiac pulsation, as well as the motion due to surgeon hand tremor. The resulting involuntary distance changes between the surgical tool and surgical tissue surface, although usually on the order of a few hundreds of micrometer at less than 5Hz, may cause serious error due to the scale of microsurgery. The "tool-tissue" relative motion is especially critical in the case of surface operations such as retina vitreous surgery and cerebral cortex neurosurgery where the fragile tissue's axial involuntary motion is a primary concern that requires high dexterity and constant attention from experienced surgeons. The following scientific article is related prior art: KANG ZHANG ET AL: "A Surface Topology and Motion Compensation System for Microsurgery Guidance and Intervention Based on Common-Path Optical Coherence Tomography",IEEE TRANSACTIONS ON BIOMEDICAL ENGINEERING, IEEE SERVICE CENTER, PISCATAWAY, NJ, USA, vol. 56, no. 9, 1 September 2009 (2009-09-01), pages 2318-2321,ISSN: 0018-9294, DOI: 10.1109/TBME.2009.2024077. Further related art is provided in the documents WO00/41611A2 and WO02/083003A1.

There thus remains a need for improved surgical tools and systems for microsurgical applications.

### SUMMARY

A motion-compensating surgical tool system according to an example includes a surgical tool that includes a hand piece and a moveable component, a drive assembly connecting the moveable component to the hand piece such that the moveable component is movable in an axial direction relative to the hand piece by the drive assembly. The motion-compensating surgical tool system also includes an optical detection system that includes an optical fiber attached to the moveable component with an end at a fixed distance to a distal-most portion of the moveable component. The optical detection system is configured to output a signal for the determination of a distance of the distal-most portion of the moveable component to a target during surgery.

A surgical tool for a motion-compensating surgical tool system according to an example includes a hand piece, a drive assembly connected to the hand piece, a moveable component connected to the drive assembly such that the moveable component is movable in an axial direction relative to the hand piece, and an optical fiber attached to the moveable component with an end at a fixed distance to a distal-most portion of the moveable component. The optical fiber is adapted to be optically coupled to an optical detection system configured to output a signal for the determination of a distance of the distal-most portion of the moveable component to a target during surgery. The invention is limited by the scope of the appended independent claim 1. Further preferred embodiments are disclosed in the dependent claims.

### BRIEF DESCRIPTION OF THE DRAWINGS

Further objectives and advantages will become apparent from a consideration of the description, drawings, and examples.
**Figure 1** is a schematic illustration of motion-compensating surgical tool system according to an embodiment of the current invention (CCD, line scan camera; G, grating; LI, L2 achromatic collimators).
**Figure 2** is a schematic illustration of a surgical tool according to an embodiment of the current invention (IN: inner needle for surgical tool mounting; ON: outer needle for protection and alignment; ONM: outer needle mount; BS: backup spring; SL: slider for inner needle mount; SQ: SQUIGGLE micro-motor; SQM: micro-motor mount; SQC: micro-motor cord; SMF: single mode).
**Figure 3A** shows a prototype of a surgical tool according to an embodiment of the current invention; **Figure 3B** shows a surgical tool tip for micro dissector; Figure 3C shows a surgical tool tip for micro forceps (SMF: single-mode fiber); **Figure 3D** shows a hand-holding scenario.
**Figures 4A-4D** illustrate an edge-searching method according to an embodiment of the current invention. **Figure 4A** shows raw A-scan data ("Ghost edge" exists). **Figure 4B** shows A-scan data after 1-D erosion algorithm ("Ghost Edge" is erased and real edge remains). **Figure 4C** shows thresholding of A-scan. **Figure 4D** shows first zero-crossing for edge location (depth scale changed).
**Figure 5A** is a system control flowchart; **Figure 5B** is a speed control curve according to an embodiment of the current invention.
**Figures 6A and 6B** show motion compensation results for an example according to an embodiment of the current invention.
**Figures 7A-7D** show an example of incision with surface tracking and motion compensation according to an embodiment of the current invention. **Figure 7A** is an en face image of the incision region; **Figure 7B** is a B-scan along the red trace in **Figure 7A** (bottom of incision); **Figure 7C** is a B-scan along the blue trace in **Figure 7A** (top of incision); and **Figure 7D** is the combination of (b) and(c).
**Figures 8A-8D** show an example of incision with free-hand. **Figure 8A** is an en face image of the incision region; **Figure 8B** is a B-scan along the red trace in **Figure 8A** (bottom of incision); **Figure 8C** is a B-scan along the blue trace in **Figure 8A** (top of incision); **Figure 8D** is a combination of (b) and(c).
**Figures 9A and 9B** show an example of incision depth change along the incision trace, with **Figure 9A** and without **Figure 9B** surface tracking and motion compensation.

### DETAILED DESCRIPTION

Some embodiments of the current invention are discussed in detail below. In describing embodiments, specific terminology is employed for the sake of clarity. However, the invention is not intended to be limited to the specific terminology so selected. A person skilled in the relevant art will recognize that other equivalent components can be employed and other methods developed without departing from the scope of the claim.

Some embodiments of the current invention can provide a simple and compact hand-held microsurgical tool capable of surface tracking and motion compensation based on a common-path optical coherence tomography (CP-OCT) distance-sensor to improve the accuracy and safety of microsurgery. Some embodiments of the current invention use a single fiber probe as a CP-OCT distance-sensor and a high-speed piezo-electric micro linear motor for the 1-D actuation. The distance between the tool tip and the surgical target surface is determined from the OCT signal by an automatic edge-searching algorithm. The target surface can be the surface of tissue, for example, or it could be within tissue. The micro linear motor is controlled by a computer according to feedback from the CP-OCT distance-sensor. In a current example, CP-OCT microsurgical tool according to an embodiment of the current invention is miniaturized into a 15 mm-diameter plastic syringe and is capable of surface tracking at less than 5 micrometer resolution. A phantom made with intralipid layer was used to simulate a real tissue surface and a single-fiber integrated micro-dissector works as a surgical tip to perform tracking and accurate incision on the phantom surface in this example.

Some embodiments of the current invention can be integrated into standard freehand microsurgical tools and enable surgeons to make precise surgical maneuvers safely and effectively.

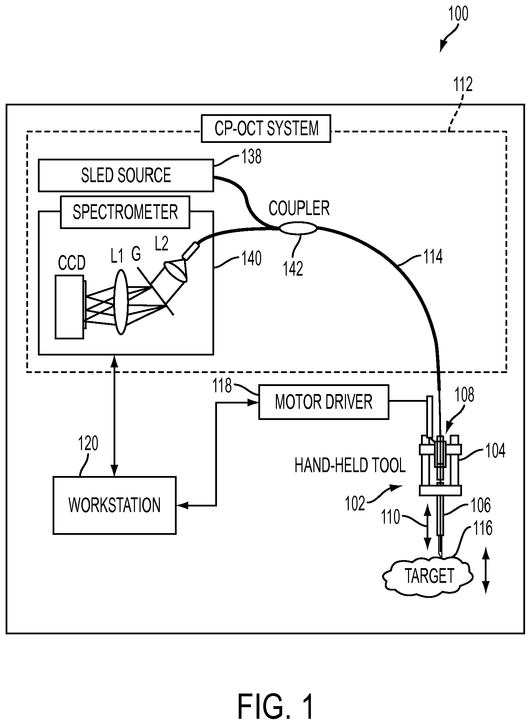

Figure 1 is a schematic illustration of a motion-compensating surgical tool system 100 according to an embodiment of the current invention. The motion-compensating surgical tool system 100 includes a surgical tool 102 that includes a hand piece 104 and a moveable component 106. The surgical tool also includes a drive assembly 108 connecting the moveable component 106 to the hand piece 104 such that the moveable component 106 is movable in an axial direction 110 relative to the hand piece 104 by the drive assembly 108. The surgical tool system 100 also includes an optical detection system 112 that includes an optical fiber 114 attached to the moveable component 106 with an end at a fixed distance to a distal-most portion of the moveable component 106. The optical detection system 112 is configured to output a signal for the determination of a distance of the distal-most portion of the moveable component 106 to a target 116 during surgery.

The motion-compensating surgical tool system 100 also includes a drive controller 118 configured to communicate with the drive assembly 108 for moving the moveable component 106 of the surgical tool 102. The motion-compensating surgical tool system 100 also includes a data processor 120 configured to communicate with the optical detection system 112 to receive the signal for the determination of the distance and to communicate with the drive controller 118.

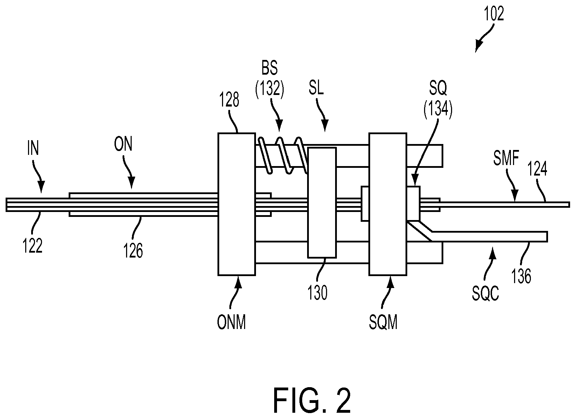

Figure 2 is a schematic illustration of an enlarged view of surgical tool 102. In this example, the moveable component 106 is an inner needle 122 that has an optical fiber 124 down the center. The optical fiber 124 can be a single mode optical fiber, for example. In this embodiment, there is also an outer needle 126 that provides protection and alignment. The outer needle 126 is held by outer needle mount 128. The inner needle 122 is attached to slider 130 with a backspring 132 connected between the outer needle mount 128 and the slider 130 to provide a restoring force. A micromotor 134 drives the slider 130 to thereby drive the inner needle 122. A SQUIGGLE micro-motor has been found to be suitable for some embodiments of the micromotor 134. The micromotor 134 has a micromotor cord 136 attached to it.

The data processor 120 can be configured to perform edge searching to determine at least one of an edge, a center of mass, or an axial feature of the target 116 according to some embodiments of the current invention. The data processor 120 can be a dedicated "hard-wired" device, or it can be a programmable device. For example, it can be, but is not limited to, a personal computer, a work station or any other suitable electronic device for the particular application. In some embodiments, it may be integrated into a unit or it could be attachable, remote and/or distributed.

In some embodiments, data processor 120 is further configured to determine an amount, speed and/or direction of movement of the moveable component 106 to be moved by the drive assembly 108 to counter motions of the distal-most portion of the moveable component 106 relative to the target 116 during surgery.

In some embodiments, the optical detection system 112 also includes a light source 138 optically coupled to the optical fiber 114 and an optical detector 140 optically coupled to the optical fiber 114. For example, a 2x1 optical coupler 142 can be included to couple the light source 138 and optical detector to the optical fiber 114. (The term "light" as used herein is intended to have a broad meaning. For example, infrared, visible and ultraviolet light can be included within the broad meaning of the term "light.") The optical fiber 114 provides a common transmit and receive optical path such that the optical detection system is a common-path optical coherence tomography system (CP-OCT).

In some embodiments, the light source 138 can be a superluminescent light emitting diode. In some embodiments, the optical detector 140 can be a spectrometer, as is illustrated in the example of Figure 1. In other embodiments, the light source 138 can be a wavelength swept laser and the optical detector 140 can be a photodetector.

In some embodiments, the drive assembly 108 can include a piezo-electric micromotor that has a traveling range of at least 20mm, a maximum speed of at least 4mm/second, and a travel resolution of at least 0.5µm. In some embodiments, the drive assembly can include a piezo-electric micromotor that weighs less than about 100 grams.

In some embodiments, the surgical tool 102 can be, but is not limited to, a micro-surgical tool. The micro-surgical tool can be one of a needle, a pick, a scalpel, forceps, scissors, or a trocar, for example.

In the example of Figure 1, a Fourier domain CP-OCT system provides the sensing part, which includes a superluminescent diode (SLED) light source, a high speed spectrometer and a broad band fiber coupler. A single-mode fiber, which is integrated in to a hand-held tool works as the distance sensing probe. The distance between the tool tip and the surgical target surface is directly determined from the OCT signal by an automatic edge-searching algorithm, and the micro linear motor is controlled by the computer according to the feedback from CP-OCT distance-sensor.

In the CP-OCT system section, a 12-bit CCD line-scan camera (e2v, EM4, USA) is used as the detector of the OCT spectrometer. A superluminescence (SLED) source (λ₀=870nm, Δλ=180nm, Superlum, Ireland) is used as the light source, giving an experimental axial resolution of 3.6µm in air. The minimum line period is camera-limited to 14.2µs, corresponding to a maximum line rate of 70k A-scan/s. A single-mode fiber which is integrated in to the hand-held tool works as the distance sensing probe. The distance between the tool tip and the surgical target surface is directly determined from the OCT signal by an automatic edge-searching algorithm (Zhang, K., Wang, W., Han, J-H., Kang, J. U., "A surface topology and motion compensation system for microsurgery guidance and intervention based on common-path optical coherence tomography," IEEE Trans. Biomed. Eng., 56(9), 2318-2321 (2009); Zhang, K., Akpek, E. K., Weiblinger, R. P., Kim, D-H., Kang, J. U., and Ilev, I. K., "Noninvasive volumetric quality evaluation of post-surgical clear corneal incision via high-resolution Fourier-domain optical coherence tomography," Electron. Lett., 46(22), 1482-1483(2010), and the micro linear motor is controlled by the computer according to the feedback from CP-OCT distance-sensor. A quad-core Dell T7500 workstation was used to host the frame grabber, motor controller and perform real-time signal processing and feedback control.

In the example of Figure 2, the hand-held microsurgical tool uses a piezoelectric micro-motor (LEGS-L01S-11, PiezoMotor AB, Sweden) as the mechanical module to drive the slider with mounted inner needle. The LEGS-L01S-11 motor has a 35 mm travel range, 20mm/s maximum speed, less than 1 nm resolution depending on different control modes, and a 10N maximum driving force. A variety of microsurgical tools such as forceps, scissors and micro-trocars, for example, can be attached to the inner needle tip and a single mode CP-OCT fiber probe can be fixed inside of the inner needle.

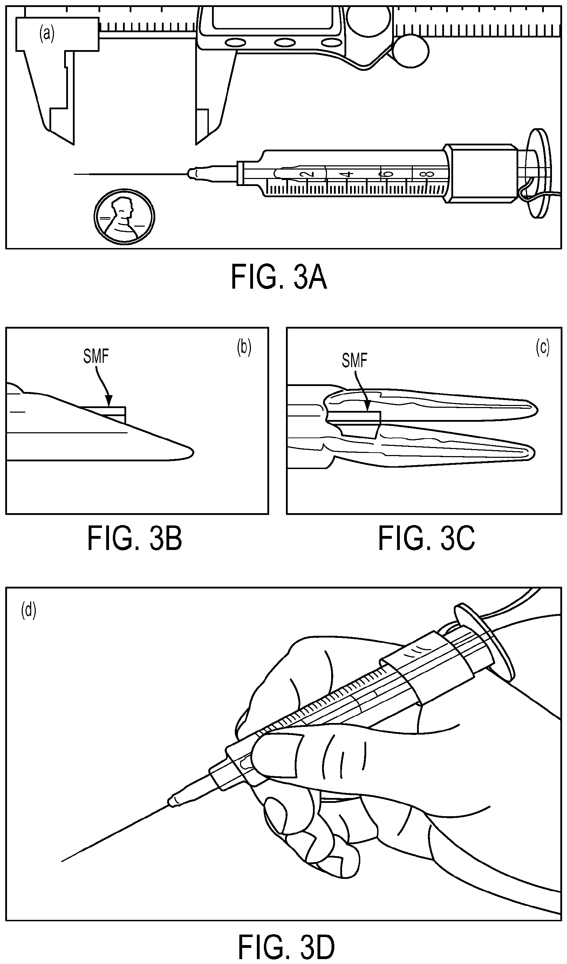

Figure 3A shows a prototype of surgical tool system, according to an embodiment of the current invention, which is integrated into a 15mm-diameter plastic syringe. Figures 3B and 3C show examples of the surgical tool tip with an integrated single mode fiber (5.6 µm /125 µm Core/Clad diameter). The fixed distance between fiber tip and surgical tool tip can be pre-measured precisely by a digital microscope. Figure 3D shows an example of a hand-holding scenario of the surgical tool prototype.

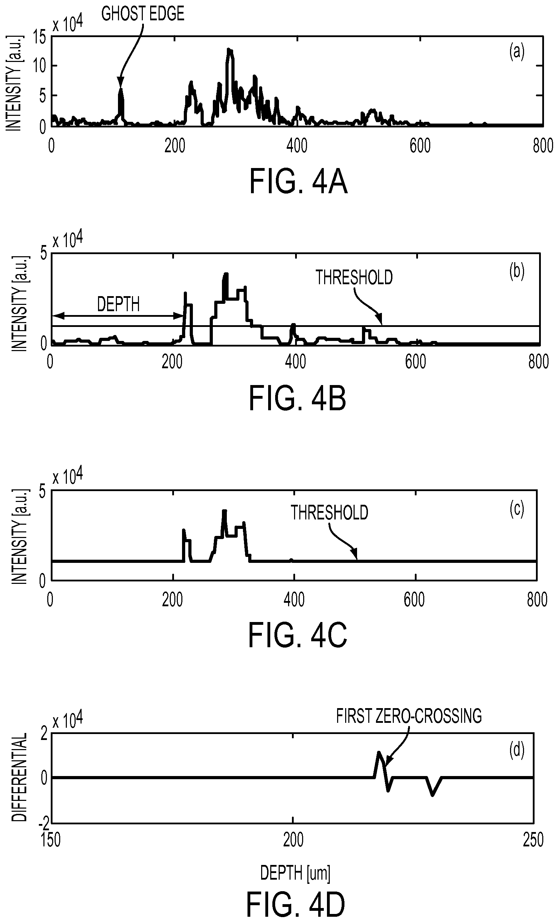

Figures 4A-4D shows an example of an edge-searching algorithm for determining the fiber tip to target surface distance from the OCT signal according to an embodiment of the current invention. The raw OCT signal is first filtered by a 1-D erosion filter to smooth the signal and remove the possible "Ghost Edge". Then the new A-scan data is processed by using a certain threshold level to avoid the noise effect. Finally the edge position is given by the first zero-crossing of the differential of the post-thresholding data.

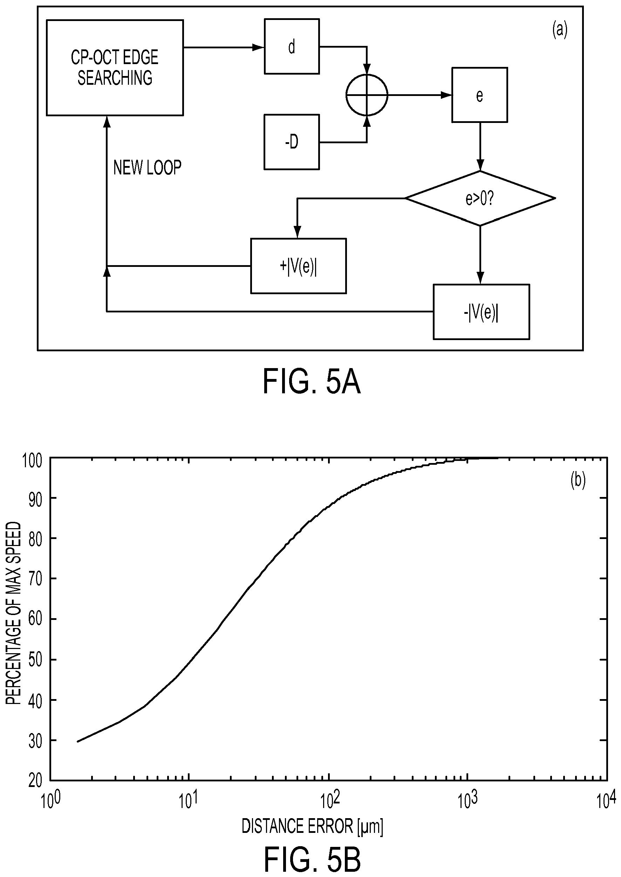

Figures 5A and 5B show a control flowchart and speed control curve, respectively, of an example of a surgical tool system according to an embodiment of the current invention. The edge searching algorithm is used to determine the CP-OCT probe-to-target distance d, which is desired to be maintained at a fixed distance D. A speed control curve V(e) is applied in the algorithm to avoid overshooting and oscillation during tracking, where e is the error between d and D.

### EXAMPLES

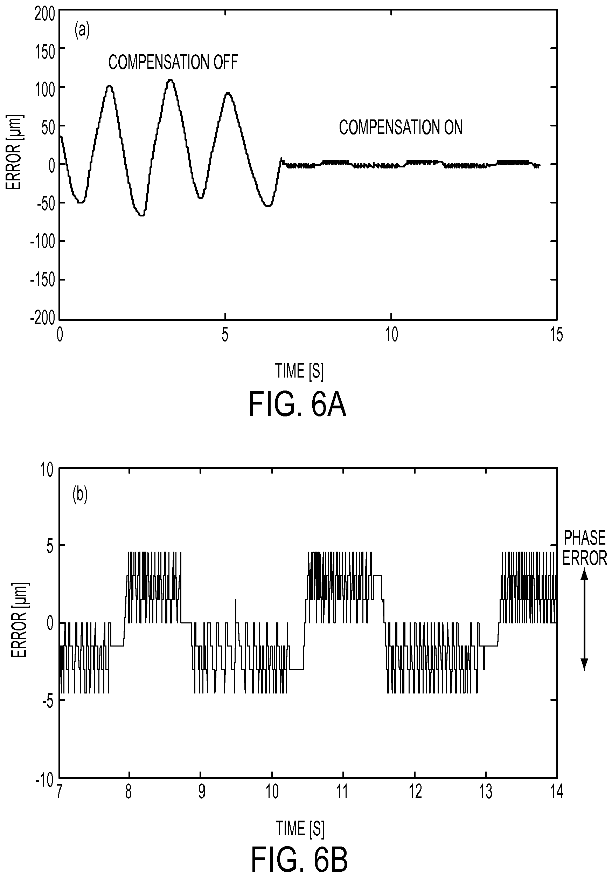

First we implemented the surface tracking and motion compensation tests for an example. The surgical tool tip is pointed perpendicularly to a reflective target surface, which moves back and forth from the initial position. The tool tip senses the motion and adjusts to keep a constant distance D=1120µm by moving together with the target surface. The error changing between d and D is recorded with time, and the compensation effect is apparent by comparing the error values between "Compensation off" mode and "Compensation on" mode, as in Figure 6A. Figure 6B shows a zoomed-in view of the error-time curve at "Compensation on" mode in Figure 6A. The compensation error is within ±5 µm, which agrees well with the resolution of CP-OCT. The phase error can be further reduced by applying a predictive filter into the tracking algorithm (Zhang, K., Wang, W., Han, J-H., Kang, J. U., "A surface topology and motion compensation system for microsurgery guidance and intervention based on common-path optical coherence tomography," IEEE Trans. Biomed. Eng., 56(9), 2318-2321 (2009)).

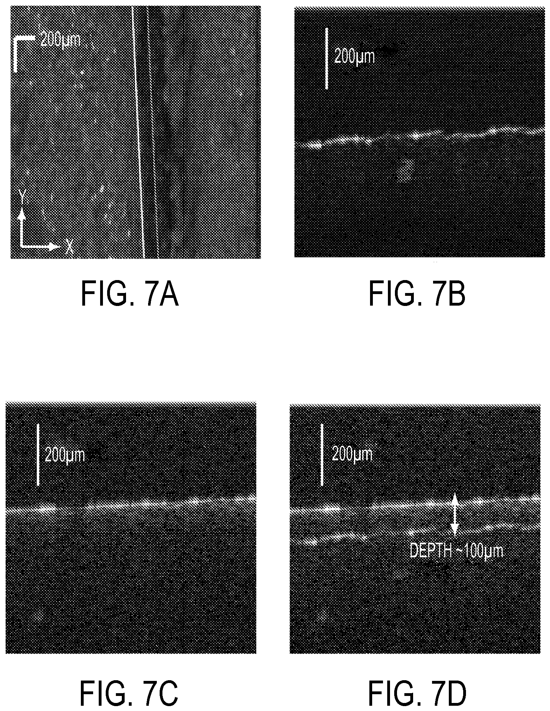

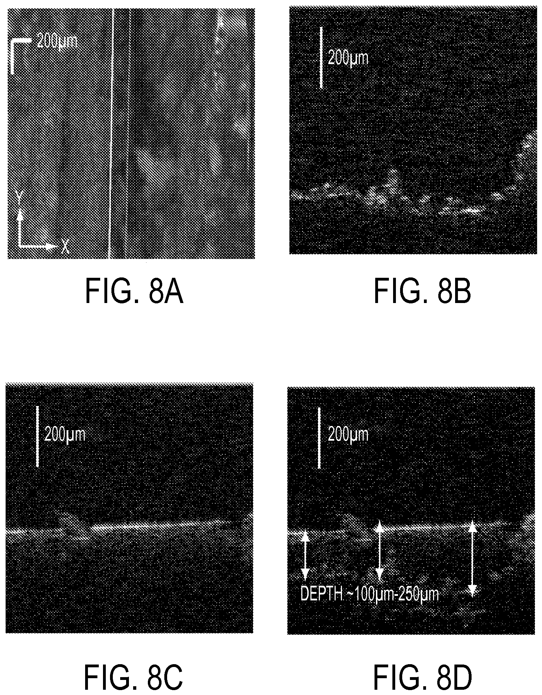

Then, to show that these tools will actually enhance a surgeon's ability, using phantoms fabricated from intralipid film, we performed surgical incisions with intended depth of 100µm with and without the surface tracking and motion compensation. To show the incision depth relative to the phantom surface, we took the 3-D volumetric OCT images of the incision sites using our high speed FD-OCT system (Zhang, K. and Kang, J. U., "Real-time 4D signal processing and visualization using graphics processing unit on a regular nonlinear-k Fourier-domain OCT system," Opt. Express, 18(11), 11772-11784 (2010)). The image volume is 1000(X)× 150(Y) ×512(Z) voxels. Figure 7A and 8A show the *en face* projections of the incision region. The blue and red lines indicate the cross-sectional imaging traces of the top edge and bottom of the incisions. As finally combined shown in Figure 7D and 8D, the incision with surface tracking and compensation has constant incision depth of about 100 µm, while the free-hand incision has an irregular depth of 100 µm to around 250 µm.

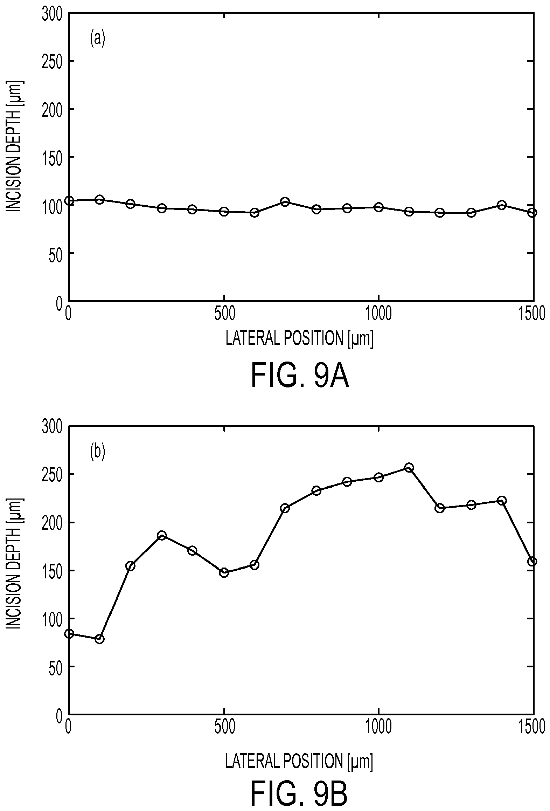

Figure 9 shows the incision depth change along the incision trace for both cases. These results clearly show that the smart tool provides highly accurate incision depth control, uniform incision depth, and virtually eliminates the hand tremor. The variations in the incision depth with and without the tracking tool control were ±5µm and ±80µm, respectively.

A hand-held microsurgical tool based on a CP-OCT distance sensor according to some embodiments of the current invention was tested. The tool is capable of tracking the tissue surface of a microsurgical target and of compensating the tool-tissue relative motion on a micrometer scale. Using a phantom model, the quality of incision depth was evaluated using 3D FD-OCT, and the results using the surface tracking and motion compensation tool show significant improvement compared to the results by free-hand. Such tools can have broad applicability in other microsurgical procedures and can improve surgical accuracy and safety.

The embodiments illustrated and discussed in this specification are intended only to teach those skilled in the art how to make and use the invention. In describing embodiments of the invention, specific terminology is employed for the sake of clarity. However, the invention is not intended to be limited to the specific terminology so selected. The above-described embodiments of the invention may be modified or varied, without departing from the invention, as appreciated by those skilled in the art in light of the above teachings. It is therefore to be understood that, within the scope of the claims and their equivalents, the invention may be practiced otherwise than as specifically described.

## Claims

1. A motion-compensating, hand-held surgical tool system (100), comprising:
a surgical tool (102), comprising:
a hand piece (104);
a drive assembly (108) connected to said hand piece (104);
a moveable component (106) connected to said drive assembly (108) such that said moveable component (106) is movable by said drive assembly (108) in an axial direction (110) relative to said hand piece (104); and
an optical fiber (114) attached to said moveable component (106) with an end at a fixed distance to a distal-most portion of said moveable component (106);
a drive controller (118) configured to communicate with said drive assembly (108) for moving said moveable component (106) of said surgical tool (102);
an optical detection system (112) coupled to said optical fiber (114);
a data processor (120) configured to communicate with said optical detection system (112),
wherein said optical detection system (112) is configured to output a signal for the determination of a distance of said distal-most portion of said moveable component (106) to a target (116) during hand-held use; and
wherein said data processor is configured to:
receive said signal for the determination of said distance of said distal-most portion of said moveable component (106) to a target (116);
calculate a difference between said determined distance and a desired distance; and
apply **an S-shaped** speed control curve based on a logarithmic scale of the distance, difference, wherein the speed control curve is dependent on the determined difference between said determined distance and said desired distance and wherein the value of speed control curve increases when said difference increases; and
communicate with the drive controller to maintain said desired distance.

2. The motion-compensating, hand-held surgical tool system (100) according to claim 1, wherein said drive assembly (108) comprises a piezo-electric micromotor, preferably wherein said piezo-electric micromotor has a traveling range of at least 20mm, at least a 4mm/second maximum speed, and a travel resolution of at least 0.5µm.

3. The motion-compensating, hand-held surgical tool system (100) according to claim 2, wherein said drive assembly (108) comprises a piezo-electric micromotor that weighs less than about 100 grams.

4. The motion-compensating, hand-held surgical tool system (100) according to claim 1, wherein said surgical tool (102) is a micro-surgical tool.

5. The motion-compensating, hand-held surgical tool system (100) according to claim 4, wherein said micro-surgical tool is one of a needle, a pick, a scalpel, forceps, scissors, or a trocar.

6. The motion-compensating, hand-held surgical tool system (100) according to claim 1,
wherein said data processor (120) is a configured to perform edge searching to determine at least one of an edge, a center of mass, or an axial feature of said target (116).

7. The motion-compensating, hand-held surgical tool system (100) according to claim 6, wherein said data processor (120) is further configured to determine an amount, speed and direction of movement of said moveable component (106) by said drive assembly (108) to counter motions of said distal-most portion of said moveable component (106) relative to said target (116) during surgery.

8. The motion-compensating, hand-held surgical tool system (100) according to claim 1, wherein said optical detection system (112) further comprises a light source (138) optically coupled to said optical fiber (114) and an optical detector (140) optically coupled to said optical fiber (114), wherein said optical fiber (114) provides a common transmit and receive optical path such that said optical detection system (112) is a common-path optical coherence tomography system.

9. The motion-compensating, hand-held surgical tool system (100) according to claim 8, wherein said light source (138) is a superluminescent light emitting diode or
a wavelength swept laser and said optical detector (140) is a photodetector or
a spectrometer.

## Patentansprüche

1. Bewegungskompensierendes handgeführtes chirurgisches Werkzeugsystem (100), umfassend:
ein chirurgisches Werkzeug (102), umfassend:
ein Handstück (104);
eine Antriebsbaugruppe (108), die mit dem Handstück (104) verbunden ist;
eine bewegliche Komponente (106), die so mit der Antriebsbaugruppe (108) verbunden ist, dass die bewegliche Komponente (106) durch die Antriebsbaugruppe (108) in axialer Richtung (110) relativ zu dem Handstück (104) beweglich ist; und
eine optische Faser (114), die mit einem Ende in einem festen Abstand zu dem distalsten Teil der beweglichen Komponente (106) an der beweglichen Komponente (106) befestigt ist;
ein Antriebssteuergerät (118), das so konfiguriert ist, dass es mit der Antriebsbaugruppe (108) kommuniziert, um die bewegliche Komponente (106) des chirurgischen Werkzeugs (102) zu bewegen;
ein optisches Nachweissystem (112), das an die optische Faser (114) gekoppelt ist;
einen Rechner (120), der so konfiguriert ist, dass er mit dem optischen Nachweissystem (112) kommuniziert;
wobei das optische Nachweissystem (112) so konfiguriert ist, dass es während der handgeführten Verwendung ein Signal für die Bestimmung eines Abstands des distalsten Teils der beweglichen Komponente (106) zu einem Target (116) ausgibt; und
wobei der Rechner konfiguriert ist, um:
das Signal für die Bestimmung des Abstands des distalsten Teils der beweglichen Komponente (106) zu einem Target (116) zu empfangen;
eine Differenz zwischen dem bestimmten Abstand und einem gewünschten Abstand zu berechnen; und
eine S-förmige Geschwindigkeitskontrollkurve auf der Basis einer logarithmischen Skala der Abstandsdifferenz anzuwenden;
wobei die Geschwindigkeitskontrollkurve von der bestimmten Differenz zwischen dem bestimmten Abstand und dem gewünschten Abstand abhängt und wobei der Wert der Geschwindigkeitskontrollkurve zunimmt, wenn die Differenz zunimmt; und
mit dem Antriebssteuergerät zu kommunizieren, um den gewünschten Abstand aufrechtzuerhalten.

2. Bewegungskompensierendes handgeführtes chirurgisches Werkzeugsystem (100) gemäß Anspruch 1, wobei die Antriebsbaugruppe (108) einen piezoelektrischen Mikromotor umfasst, wobei vorzugsweise der piezoelektrische Mikromotor einen Bewegungsbereich von wenigstens 20 mm, eine Maximalgeschwindigkeit von wenigstens 4 mm/Sekunde und eine Bewegungsauflösung von mindestens 0,5 µm aufweist.

3. Bewegungskompensierendes handgeführtes chirurgisches Werkzeugsystem (100) gemäß Anspruch 2, wobei die Antriebsbaugruppe (108) einen piezoelektrischen Mikromotor umfasst, der weniger als etwa 100 Gramm wiegt.

4. Bewegungskompensierendes handgeführtes chirurgisches Werkzeugsystem (100) gemäß Anspruch 1, wobei das chirurgische Werkzeug (102) ein mikrochirurgisches Werkzeug ist.

5. Bewegungskompensierendes handgeführtes chirurgisches Werkzeugsystem (100) gemäß Anspruch 4, wobei das mikrochirurgische Werkzeug aus einer Nadel, einer Pinzette, einem Skalpell, einer Zange, einer Schere oder einem Trokar ausgewählt ist.

6. Bewegungskompensierendes handgeführtes chirurgisches Werkzeugsystem (100) gemäß Anspruch 1, wobei der Rechner (120) so konfiguriert ist, dass er eine Randsuche durchführt, um wenigstens eines aus einem Rand, einem Schwerpunkt oder einem axialen Merkmal des Targets (116) zu bestimmen.

7. Bewegungskompensierendes handgeführtes chirurgisches Werkzeugsystem (100) gemäß Anspruch 6, wobei der Rechner (120) weiterhin so konfiguriert ist, dass er die Menge, die Geschwindigkeit und die Richtung der Bewegung der beweglichen Komponente (106) durch die Antriebsbaugruppe (108) bestimmt, um Bewegungen des distalsten Teils der beweglichen Komponente (106) relativ zu dem Target (116) während einer Operation entgegenzuwirken.

8. Bewegungskompensierendes handgeführtes chirurgisches Werkzeugsystem (100) gemäß Anspruch 1, wobei das optische Nachweissystem (112) weiterhin eine Lichtquelle (138), die optisch an die optische Faser (114) gekoppelt ist, und einen optischen Detektor (140), der optisch an die optische Faser (114) gekoppelt ist, umfasst, wobei die optische Faser (114) für einen gemeinsamen Sende- und Empfangsweg sorgt, so dass das optische Nachweissystem (112) ein Common-Path-OCT(optische-Kohärenz-Tomographie)-System ist.

9. Bewegungskompensierendes handgeführtes chirurgisches Werkzeugsystem (100) gemäß Anspruch 8, wobei die Lichtquelle (138) eine Superlumineszenzdiode oder ein wellenlängenvariabler Laser ist und der optische Detektor (140) ein Photodetektor oder ein Spektrometer ist.

## Revendications

1. Système d'outil chirurgical manuel à compensation de mouvement (100), comprenant :
un outil chirurgical (102), comprenant :
une pièce à main (104) ;
un ensemble d'entraînement (108) raccordé à ladite pièce à main (104) ;
un composant mobile (106) raccordé audit ensemble d'entraînement (108) de sorte que ledit composant mobile (106) soit mobile par ledit ensemble d'entraînement (108) dans une direction axiale (110) par rapport à ladite pièce à main (104) ; et
une fibre optique (114) attachée audit composant mobile (106) avec une extrémité à une distance fixe d'une portion la plus distale dudit composant mobile (106) ;
un dispositif de commande d'entraînement (118) configuré pour communiquer avec ledit ensemble d'entraînement (108) pour déplacer ledit composant mobile (106) dudit outil chirurgical (102) ;
un système de détection optique (112) couplé à ladite fibre optique (114) ;
un processeur de données (120) configuré pour communiquer avec ledit système de détection optique (112),
dans lequel ledit système de détection optique (112) est configuré pour fournir en sortie un signal pour déterminer une distance de ladite portion la plus distale dudit composant mobile (106) jusqu'à une cible (116) pendant ladite utilisation manuelle ; et
dans lequel ledit processeur de données est configuré pour :
recevoir ledit signal pour déterminer ladite distance de ladite portion la plus distale dudit composant mobile (106) jusqu'à une cible (116) ;
calculer une différence entre ladite distance déterminée et une distance souhaitée ; et
appliquer une courbe de commande de vitesse en S d'après une échelle logarithmique de la différence de distance,
dans lequel la courbe de commande de vitesse dépend de la différence déterminée entre ladite distance déterminée et ladite distance souhaitée et dans lequel la valeur de la courbe de commande de vitesse augmente lorsque ladite différence augmente ; et
communiquer avec le dispositif de commande d'entraînement pour maintenir ladite distance souhaitée.

2. Système d'outil chirurgical manuel à compensation de mouvement (100) selon la revendication 1, dans lequel ledit ensemble d'entraînement (108) comprend un micromoteur piézoélectrique, dans lequel de préférence ledit micromoteur piézoélectrique a une plage de course d'au moins 20 mm, une vitesse maximale d'au moins 4 mm/seconde, et une résolution de course d'au moins 0,5 µm.

3. Système d'outil chirurgical manuel à compensation de mouvement (100) selon la revendication 2, dans lequel ledit ensemble d'entraînement (108) comprend un micromoteur piézoélectrique qui pèse moins d'environ 100 grammes.

4. Système d'outil chirurgical manuel à compensation de mouvement (100) selon la revendication 1, dans lequel ledit outil chirurgical (102) est un outil microchirurgical.

5. Système d'outil chirurgical manuel à compensation de mouvement (100) selon la revendication 4, dans lequel ledit outil microchirurgical est l'un d'une aiguille, d'une pointe, d'un scalpel, d'un forceps, de ciseaux, ou d'un trocart.

6. Système d'outil chirurgical manuel à compensation de mouvement (100) selon la revendication 1, dans lequel ledit processeur de données (120) est configuré pour réaliser une recherche de bord pour déterminer au moins l'un d'un bord, d'un centre de masse ou d'une particularité axiale de ladite cible (116).

7. Système d'outil chirurgical manuel à compensation de mouvement (100) selon la revendication 6, dans lequel ledit processeur de données (120) est en outre configuré pour déterminer une quantité, une vitesse et une direction de déplacement dudit composant mobile (106) par ledit ensemble d'entraînement (108) pour contrer des mouvements de ladite portion la plus distale dudit composant mobile (106) par rapport à ladite cible (116) pendant une opération.

8. Système d'outil chirurgical manuel à compensation de mouvement (100) selon la revendication 1, dans lequel ledit système de détection optique (112) comprend en outre une source de lumière (138) couplée optiquement à ladite fibre optique (114) et un détecteur optique (140) couplé optiquement à ladite fibre optique (114), dans lequel ladite fibre optique (114) fournit un chemin optique de transmission et de réception commun de sorte que ledit système de détection optique (112) soit un système de tomographie par cohérence optique de chemin commun.

9. Système d'outil chirurgical manuel à compensation de mouvement (100) selon la revendication 8, dans lequel ladite source de lumière (138) est une diode superluminescente ou un laser à balayage de longueur d'onde et ledit détecteur optique (140) est un photodétecteur ou un spectromètre.
